# EUROPEAN PATENT SPECIFICATION

(11) **EP 3 193 778 B1**
(45) Date of publication and mention of the grant of the patent: **06.05.2020**
(21) Application number: 15840142.2
(22) Date of filing: 14.09.2015
(51) Int. Cl.: A61F 2/01

(54) **INTRODUCER SHEATH WITH EMBOLIC PROTECTION**
EINFÜHRUNGSSCHLEUSE MIT EMBOLIESCHUTZ
GAINE D'INTRODUCTION À PROTECTION EMBOLIQUE

(30) Priority: 14.09.2014 US 201462050156 P; 10.11.2014 US 201414537814
(43) Date of publication of application: 26.07.2017
(60) Divisional application: 20164920.9
(73) Proprietor: Emboline, Inc., Santa Cruz, CA 95060 (US)
(72) Inventor: RUSSELL, Scott, M., Santa Cruz, CA 95060 (US); BELSON, Amir, Cupertino, California 95014 (US)
(74) Representative: Clark, Jane Anne
(86) International application number: PCT/US2015/049908
(87) International publication number: WO 2016/040923

(56) References cited:
- WO-A2-2004/021922
- WO-A2-2006/138391
- CA-A1- 2 609 800
- US-A1- 2006 287 668
- US-A1- 2012 016 408
- US-A1- 2013 096 606
- US-A1- 2013 178 891
- US-A1- 2013 178 891
- US-A1- 2013 245 669
- US-A1- 2014 249 568

## Description

### BACKGROUND OF THE INVENTION

1. Field of the Invention. The present invention relates generally to medical devices and more particularly to devices for providing embolic protection to a patient's aortic arch vessels during cardiac surgery and interventional cardiology procedures.

Cerebral embolism is a known complication of cardiac surgery, cardiopulmonary bypass and catheter-based interventional cardiology and electrophysiology procedures. Embolic particles, which may include thrombus, atheroma, and lipids, may become dislodged by surgical or catheter manipulations and enter the bloodstream, embolizing in the brain or other vital organs downstream. Cerebral embolism can lead to neuropsychological deficits, stroke and even death.

Prevention of embolism would benefit patients and improve the outcome of many surgical procedures. Many current devices for preventing cerebral embolism may be less than ideal in various respects. For example, such current devices may involve multiple components and multiple steps, making the use of such devices cumbersome and even injury-prone for the patient. Also, when used with other catheter-based interventional tools, the patient's vasculature may need to be accessed at multiple points and through multiple paths. For example, a current embolic protection device may be advanced into the aortic arch through the descending aorta while other catheter-based interventional tools may then need to be advanced into or into proximity with the heart through other blood vessels including the vena cava, the right common carotid artery, and the left common carotid artery.

U.S. Patent Publ. 2013/0178891, describes an embolic protection device having embolic protection elements that are combined with an access sheath suitable advancing a diagnostic catheter. The embolic protection elements include an embolic filter slidably mounted on a distal portion of the sheath, a proximal stop for limiting the proximal movement of the embolic filter, and a distal stop for limiting the distal movement of the embolic filter. The filter comprises a porous mesh material defining a collection chamber for captured emboli and has a collapsed and a deployed configuration. The filter may be collapsed by an access sheath used with the catheter. An access sheath may comprise a tubular main body and an embolic filter mounted on the distal portion of the tubular main body. The embolic filter may revert into the central lumen of the sheath or may be constrained on the exterior of the sheath with a larger diameter outer tube.

While very effective for protecting the aortic branch vessels from emboli, the illustrated access sheath allows only a single catheter at a time to pass through the filter. Moreover, the sheath dimensions can limit the size of catheter which can be introduced which is a particular concern if a valvuloplasty catheter or prosthetic aortic or other valve is to be delivered over the aortic arch to the heart. Therefore, improved devices, systems, and methods for preventing embolism during cardiac procedures performed over the aortic arch that overcome at least some of the aforementioned short-comings are desired.

2. Description of the Background Art. U.S. Patent Publ. 2013/0178891 has been described above. Other devices for capturing or blocking emboli to prevent cerebral embolism are described in the following patent application and patent publications: U.S. Pub. No. 2010/0312268 to Belson, entitled "Embolic Protection Device"; U.S. Pub. No. 2004/0215167 to Belson, entitled "Embolic Protection Device"; U.S. Pub. No. 2003/0100940 to Yodfat, entitled "Implantable Intraluminal Protector Device and Method of Using Same for Stabilizing Atheromoas"; PCT Pub. No. WO/2004/019817 to Belson, entitled "Embolic Protection Device"; U.S. Pat. No. 6,537,297 to Tsugita et al., entitled "Methods of Protecting a Patient from Embolization During Surgery"; U.S. Pat. No. 6,499,487 to McKenzie et al., entitled "Implantable Cerebral Protection Device and Method of Use"; U.S. Pat. No. 6,371,935to Macoviak et al., entitled "Aortic Catheter with Flow Divider and Methods for Preventing Cerebral Embolization"; U.S. Pat. No. 6,361,545 to Macoviak et al., entitled "Perfusion Filter Catheter"; U.S. Pat. No. 6,254,563 to Macoviak et al., entitled "Perfusion Shunt Apparatus and Method"; U.S. Pat. No. 6,139,517 to Macoviak et al., entitled "Perfusion Shunt Apparatus and Method"; and U.S. Pat. No. 5,769,819 to Barbut et al., entitled "Cannula with Associated Filter."

### BRIEF SUMMARY OF THE INVENTION

The present invention is set out in the appended claims. Described herein are methods, systems, and devices for collecting emboli and in particular for preventing the release of emboli into the cerebral vasculature during the performance of interventional procedures in a patient's aorta, including aortic valve replacement, aortic valve valvuloplasty, and the like, where there is a risk of emboli being released into the aortic side vessels, including the brachiocephalic artery, the left carotid artery, and the left subclavian artery. The present disclosure provides an embolic protection device and system which can be placed through the descending aorta and over the aortic arch to inhibit emboli release into the aortic side branch vessels while allowing simultaneous access to the aortic valve by at least two interventional and/or diagnostic catheters being introduced from the descending aorta, typically by conventional femoral artery access.

The embolic protection device will include both an embolic filter and an inner sheath connected to the embolic filter. The inner sheath is attached to or on a downstream portion of the embolic filter, where downstream refers to the direction towards the descending aorta and away from the heart and aortic arch. The inner sheath has a lumen and will provide a first access route to an interior of the embolic filter for introducing one diagnostic or interventional catheter at a time. At least one additional port will be formed in the embolic filter for introducing at least one additional catheter so that the additional or second catheter can be present within the interior of the embolic filter simultaneously with the initial or first catheter introduced through the sheath. The additional port will typically have an expandable diameter so it will remain generally closed when no catheter is present but will be able to open and to conform to catheters of different diameters as they are introduced therethrough into the interior of the embolic filter and typically onward to the aortic valve for performing an aortic intervention.

In a first specific aspect of the present disclosure an embolic protection device comprises an inner sheath having a lumen with a distal opening and an embolic filter. The embolic filter comprises a porous mesh material having a cylindrical outer wall which defines an interior which includes a collections chamber for capturing emboli. The filter has an open upstream end and a closed downstream end, where blood and emboli my enter through the open upstream end and deposit within the collection chamber which is at least partially defined by the closed downstream end. The filter will further have both a radially collapsed delivery configuration and a radially expanded configuration, and the outer wall will typically be configured to contact a blood vessel wall to direct blood flow through the upstream end and emboli into the collection chamber. The embolic filter will have at least a first port comprising an expandable opening configured to conform to an outer wall of a first catheter passing there through and a second port which is attached to the inner sheath to allow a second catheter to be advanced through the lumen of the inner sheath so that it can enter the interior of the embolic filter.

The first port and the second port are formed in the closed downstream end of the embolic filter.

In other embodiments of the embolic protection device, the embolic filter further includes at least a first conical inner portion which defines the collection chamber between an inner surface of the outer cylindrical wall and an outer surface of the conical inner portion. In such embodiments, an apical end of the conical inner portion will be oriented towards the open upstream end of the embolic filter. In a specific aspect of the present disclosure, the first port having an expandable opening will be formed at or in the apical end of the conical portion, in this arrangement, the conical portion will have a wide opening at its downstream end to facilitate entry of a diagnostic or interventional catheter through the expandable port. The expandable port may comprise a simple slit or duck-bill-like opening, or optionally may further comprise a resilient seal positioned within or over the port for conforming to a catheter as it passes through the port.

In still further embodiments, the embolic protection device may include a second conical inner portion which, together with the first conical inner portion, will define the collection chamber between the inner surface of the cylindrical outer wall and the outer surfaces of both the first and second conical inner portions. In such instances, the apical end of the second conical inner portion will be oriented towards the upstream end of the embolic filter, and typically the inner sheath is attached to the apical end of the second conical inner portion, more typically being attached so that an upstream end of the inner sheath will be positioned beyond the apical end of the second conical structure in the upstream direction.

In still other embodiments, a distal portion of the inner sheath may pass through the second port of the embolic filter and extend in an upstream direction some distance within the interior of cylindrical wall. In some cases, the inner sheath may be attached to the cylindrical wall or, in other cases, to a side wall of the first conical inner portion. In still other embodiments, the inner sheath may pass through a wall of the first conical inner portion so that the sheath enters the embolic filter through the open downstream end of the conical inner portion and then passes into the interior of the filter portion through the wall of the conical inner portion. A variety of other ways for attaching the inner sheath to the embolic filter described in more detail below.

The porous mesh material will typically be formed from an elastic or super elastic metal, such as nickel-titanium alloy, which can be preformed into its radially expanded configuration and then constrained into its radially collapsed delivery configuration, either by an external outer delivery sheath or by an internal stylet used to elongate the embolic filter. Other available materials for the porous mesh included knitted fabrics, woven fabrics, woven fibers, non-woven fibers, filaments, and wires having a pore size chosen to prevent emboli larger than a predetermined size from passing through the mesh. Other materials include other metals, polymer materials, plastically deformable materials, and the like. In the case of malleable and plastically deformable materials, further structure may be provided to radially expand and radially collapse the embolic filter before delivery and deployment. Typical pore sizes for the mesh materials are in the range from about 0.1 mm to about 1 mm, and the porous mesh material will typically be coated with an anti-thrombogenic coating. Radiopaque markers will be typically provided on the embolic filter and/or the inner sheath.

In a second aspect of the present disclosure, a method for advancing a catheter over an aortic arch having aortic side vessels comprises providing an embolic protection device including a cylindrical outer sleeve formed at least partly from a porous mesh and having an interior which defines a collection chamber for captured emboli. The embolic protection filter will have an open upstream end, a closed downstream end, a radially collapsed delivery configuration, and a radially expanded configuration for deployment within the aortic arch. The cylindrical outer sleeve is radially expanded so that the porous mesh covers the aortic side vessels and the upstream faces the heart to direct blood flow through the upstream end of the filter and emboli into the collection chamber. As a result, blood flowing into the aortic side vessels will pass through the porous mesh which will separate the emboli. After the filter is in position, a first catheter may be advanced from an arterial lumen downstream of the closed downstream end of the embolic protection filter through a first port therein. A second catheter may be advanced from the same or a different arterial lumen downstream of the closed downstream end of the embolic protection filter through a second port. In this way, at least two catheters may be simultaneously introduced into the interior of the filter and optionally beyond to the aortic valve in order to perform the desired aortic valve interventions. For example, a small catheter for delivering contrast media may be introduced through one of the ports while a second interventional catheter may be delivered through the other port. The contrast delivery catheter may be positioned within the filter to release contrast media, while the interventional catheter may be advanced out through the open upstream end of the filter in order to perform the desired intervention on the aortic valve.

In a first specific embodiment of the methods of the present disclosure, the embolic protection filter may further include a first conical inner structure formed through the cylindrical outer sleeve and having an apical end directed into the blood flow from the heart. The first port may be disposed at or near the apical end of the first conical inner structure where the availability of the enlarged downstream end of the conical port is particularly advantageous for receiving larger, interventional catheters therethrough.

In still other embodiments, a second inner sheath may be attached to the second port so that the second catheter may be advanced through a lumen of the inner sheath before passing through the second port. The inclusion of the inner sheath provides a number of advantages. As a first advantage, the inner sheath may be used to advance and position the embolic protection filter within the aortic arch. For example, the inner sheath may be used to advance the embolic protection filter through an outer delivery sheath that constrains the embolic protection filter and its radially collapsed configuration while it is being delivered. The embolic protection filter will typically be self-expanding, as described above, allowing it to radially expand and assume its deployed configuration as it is advanced beyond a distal end of the delivery sheath. Alternatively, a stylet may be positioned within the inner sheath and extend through the embolic protection filter to selectively elongate and radially collapse the embolic protection filter while it is being delivered. When using the stylet, the embolic protection filter may be radially expanded by proximally retracting the stylet relative to the filter to release the filter from radial constraint.

The embolic protection device will usually be self-supporting in the deployed condition. In other embodiments, however, the filter can include one or more "stent-like" support structures that may comprise, for example, a framework having one or more longitudinal struts or hoops that form a an outer surrounding and/or inner supporting lattice structure that assists in the expansion and wall apposition of the device. The hoops and struts may be made of a resilient metal and/or polymer material to make a self-expanding framework or a malleable or plastically deformable material to make a framework that can be expanded with an inflatable balloon or other expansion mechanism. Alternatively, the framework can be made of a shape-memory material that can be used to deploy and/or retract the embolic protection device.

The length of embolic protection device of the present disclosure should be sufficient to cover the aortic side vessels and to extend sufficiently into the ascending and descending regions of the aorta on both sides of the side vessels to assure that no emboli can bypass the filter. Beyond that requirement, the length of the device is not critical and may be constructed with the filter mesh structure and optionally the "stent-like" support structure made either longer or shorter without adversely affecting the performance of the product. In other alternate construction, the "stent" support structure need not be cylindrical can for example be made slightly conical with the wider end of the cone oriented toward the upstream direction.

The embolic protection device of the present disclosure may be retracted and withdrawn together with or after the catheters used to perform a diagnostic or interventional procedure at the end of the procedure. Optionally, the embolic protection device may include features to assist in retracting the device for retrieval from the vessel. In one embodiment, a conical guiding structure may be slidably attached to the catheter at the proximal end of the device, the purpose of which is to assist the embolic protection device in collapsing when a retrieval sheath is advanced along the conical guiding structure. In another embodiment, portions of the embolic protection device may be constructed with retraction members or retrieval wires that are configured like purse strings or lassos around the circumference of the device. A pull loop or other graspable structure near the downstream end of the embolic protection device may be connected to the retraction members by one or more connecting members.

In still further embodiments, the filter may contain one or more support structures or wires that provide longitudinal stiffness to the device to prevent compression or movement of the filter during the procedure. Such wires or structures may extend the full length of the device or only for a portion of its length and such wires or structures shall be either fixedly or slidably attached to the access sheath.

### BRIEF DESCRIPTION OF THE DRAWINGS

Fig. 1 illustrates an exemplary embodiment of an embolic protection device constructed in accordance with the principles of the present disclosure and including an embolic filter attached to an inner sheath through the wall of a conical inner portion. The filter element is shown in an axial configuration (full line) and a curved configuration (broken line).
Figs. 2A through 20 illustrate alternative constructions for the attachment of the inner lumen to the embolic filter of the embolic protection device of Fig. 1.
Fig. 3 illustrates use of a stylet for axially elongating the embolic filter of the embolic protection device of Fig. 1 to reduce the filter diameter to a radially collapsed diameter for delivery.
Fig. 4 illustrates use of an outer sheath for a radially constraining the embolic filter of the embolic protection device of Fig. 1 for delivery.
Figs. 5A through 5E show an exemplary embolic filter deployment protocol using the embolic protection device and stylet of Figs. 1 and 3.

### DETAILED DESCRIPTION

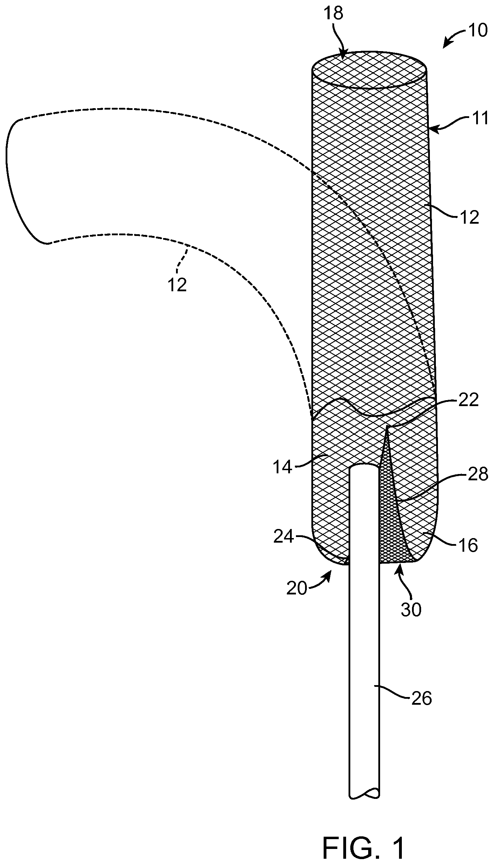

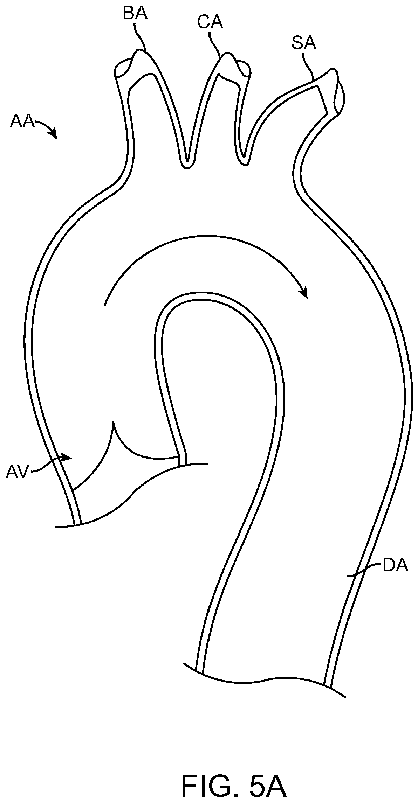

Referring to Fig. 1, an embolic protection device 10 comprises an embolic filter component 11
including a cylindrical outer wall 12, an interior space or volume 14, a collection chamber 16, and a closed downstream end 20. An open upstream end 18 lies at the opposite end of the embolic filter component 11, where the terms downstream and upstream refer to the direction when the embolic filter 11 is placed in a patient's aorta. The open upstream 18 will be facing toward the heart and thus will be facing into blood flow from the heart. The downstream end 16 will be more remote from the heart and typically be disposed in the descending aorta, as will be described in more detail below with respect to Fig. 5A.

The cylindrical wall 12 will typically be a single layer or multiple layer porous mesh, usually formed from elastic wires, filaments, tapes, and most usually being formed from super elastic wires woven into a porous structure having a pore size typically in the range from 0.1 mm to 1 mm, usually from 0.1 mm to 0.2 mm.

In preferred constructions of the embolic filter 11 and cylindrical outer wall 12, the porous mesh will be elastic and pre-formed in a radially expanded configuration (so that it can be delivered in a radially constrained configuration and released from constraint to self-expand at the location of implantation in the target blood vessel) which is somewhat larger than a target blood vessel, typically larger than a target aorta, thus usually having a diameter in the range from 15 mm to 60 mm when unconstrained, more usually having a diameter in the range from 25 mm to 45 mm when unconstrained.

The embolic filter component 11 will also have a radially collapsed delivery configuration with a reduced diameter or profile kind of typically in the range from 2 mm to 6 mm, preferably in the range from 2 mm to 4 mm. The porous mesh construction is particularly suitable for axially elongating and radially collapsing the embolic filter component, and thus a preferred construction will be a woven mesh which is otherwise minimally supported or unsupported by any other structure. In other embodiments, however, it would be possible to provide either additional or internal support structures, such as stents, scaffolds, struts, grafts, coatings, circumscribing rings, or the like, depending on the desired specific mechanical characteristics. For the most part, however, such additional structural support will be unnecessary as the woven porous mesh structure when radially expanded will have sufficient hoop strength and column strength to both deploy and be maintained within the aorta arch, as described in more detail below.

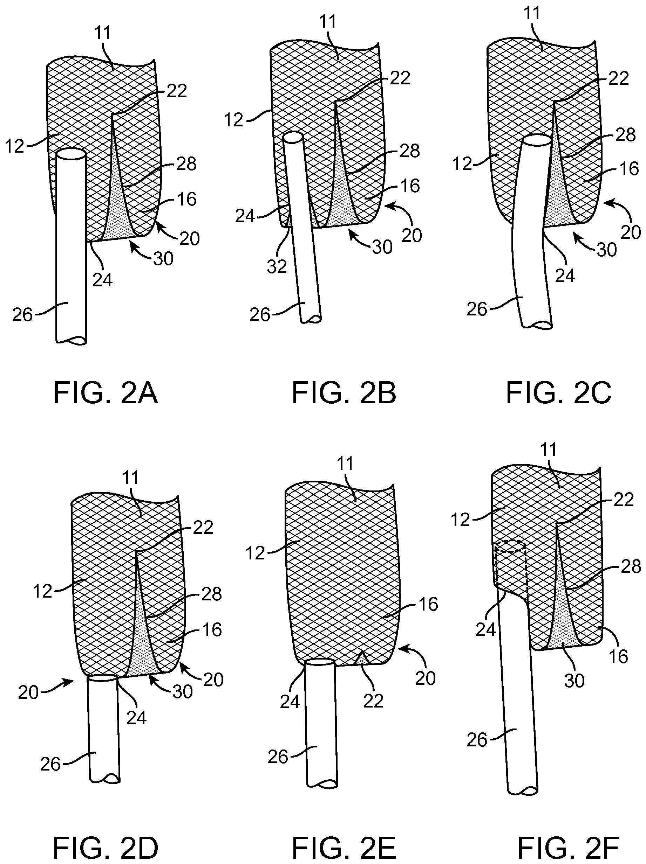

The embolic filter component 11 of the embolic protection device 10 will also include at least a first port and a second port to allow catheter access from the outside of the embolic filter component to the inside of the embolic filter component. The first port and the second port will be located on or near the closed distal end 20 of the embolic filter component.
Various specific implementations are illustrated in Figs. 2A-2F discussed here and after.

In Fig. 1, the first port 22 is formed in a first conical inner portion 28 located at the closed downstream end 20 of the embolic filtered component 11. The first conical inner portion 28 advantageously defines the collection chamber 16 immediately above the closed downstream end 20 of the embolic filter component 11 and elevates the port 22 above the bottom of the collection chamber. In this way, emboli will collect in the collection chamber 16 at the base of the conical inner portion 28, leaving the port itself free from collected emboli and reducing the chance that such emboli will pass through the port as catheters are introduced and removed there through.

In Fig. 1, the second port 24 is conveniently formed in a side wall of the first conical inner portion 28, and the inner sheath 26 is disposed within and secured to the second port. Thus, in this embodiment, catheter access through the second port 24 is achieved by passing the catheter through a lumen of the inner sheath 26. Both the first port 22 and then the inner sheath 26 open into the interior 14 of the embolic filter component 11, with the point of entry being raised above the bottom of the collection chamber 16 to reduce the risk of emboli accidently passing through either port. The first port 22 will be expandable so that, in the absence of a catheter passing there through, the port will be closed and emboli passing into the emboli filter component 11 will not be able to pass through the port. Interventional or other catheters (not pictured in Fig. 1) may, however, be passed through the first port 22 by entering through the wide opening 30 at the base of the first conical inner portion 28 and then passing through the port 22 as the catheter advances into the tapered region of the conical inner portion. A second catheter may be passed through the lumen of the inner sheath 26 in a generally conventional manner. While the lumen of the inner sheath 26 is open and thus may allow entry of embolic material, inner sheath 26 provides a closed path to its external entry port, typically through an introducer sheath into the parent patient's femeral artery, so any emboli which enter the sheath will not be released into the patient's arterial circulation.

As shown in Fig. 1, the cylindrical side wall 12 of the embolic filter component 11 will typically be straight in its unconstrained or "shelf' condition. When placed in the aortic arch or other constraint, however, the cylindrical wall 12 may be curved, e.g. as shown in broken line in Fig. 1. Thus, after deployment in the aortic arch, the outer surface of the cylindrical wall can expand into and conform to an inner wall of the aortic arch in order to provide the desired filtering of the aortic side vessels.

Referring now to Figs. 2A-2G, the downstream half of the embolic filter component 11 may have a wide variety of configurations to provide the required first and second ports. As shown in Fig. 2A, the inner sheath 26 may be passed through the closed downstream end 20 of the embolic filter component 11 at a location spaced laterally or radially from the base of the first conical inner portion 28. Instead of being attached to the side of the first conical inner portion 28, as with the embodiment of Fig. 1, a distal region of the inner sheath 26 may be attached to an inner surface of the side wall 12.

Referring now to Fig. 2B, the second port 24 may be formed in an apical region of a second conical inner portion 32. Unlike the first conical inner portion 28, however, the access sheath 26 will usually be fixedly or permanently attached to the port 24. As shown in Fig. 2B, the open distal end of the access sheath 26 extends well beyond the port 24. In other embodiments, however, the height or length of the second conical inner portion 32 could be attached directly to the second port 24.

Referring now to Fig. 2C, in a fourth specific embodiment, the inner sheath 26 may be passed through the closed downstream end 20 at location immediately adjacent to the base of the first conical inner portion 28. A distal region of the inner sheath 26 may then be attached to an outer wall portion of the first conical inner portion, as illustrated.

In a still further embodiment, as illustrated in Fig. 2D, a distal of the inner sheath 26 may be attached directly to a second port 24 formed in the closed downstream end 20 of the embolic filter component 11.

In another embodiment, as illustrated in Fig. 2E, the inner sheath 26 is attached as shown in Fig. 2D and the first port 22 is also formed directly in the closed downstream end 20 of the embolic filter component 11. The port 22 may extend minimally or not at all over a base of the closed downstream end 20.

In a still further embodiment of the inner sheath attachment detail, as shown in Fig. 2F, the inner sheath 26 may enter through a port 24 formed in the side wall 12 of the embolic filter component 11. Other aspects of the embolic filter component 11 remain the same as described in, for example, Fig. 2A above.

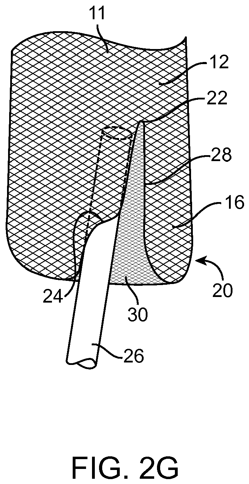

As a final exemplary embodiment of the inner sheath attachment detail as illustrated in Fig. 2G, the inner sheath 26 may enter the wide downstream end 30 of the inner conical portion 28. A port 24 is located in the region between the closed downstream end 20 and the apical end of the conical inner portion. A distal region of the inner sheath 26 may then be attached to an outer wall portion of the first conical inner portion 28. Having the distal region of the inner sheath 26 pass through the inner wall of the conical inner portion provides a particularly secure connection between the sheath and the filter.

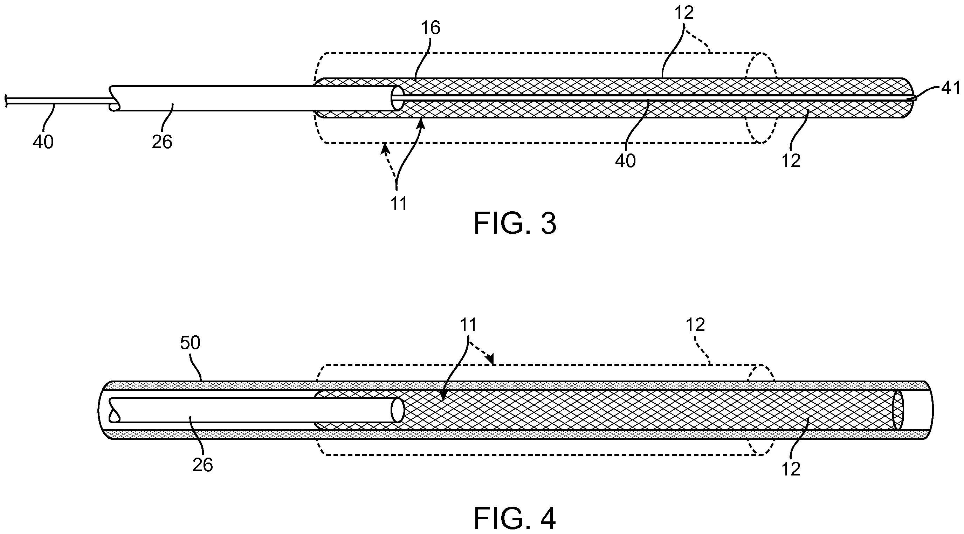

Referring now to Fig. 3, the embolic filter component 11 may be configured to have a reduced diameter for delivery by inserting a stylet 40 into the interior 16 of the embolic filter component 11 so that a distal tip 41 of the stylet engages the downstream end of the embolic filter and axially elongates the filter in order to radially collapse the diameter, typically to a diameter in the ranges as set forth above. As shown in Fig. 3, the cylindrical wall 12 of the embolic filter component 11 is shifted from an unconstrained diameter, shown in broken line, to the radially collapsed diameter, as shown in full line. In a further embodiment, to enable a more secure connection, the stylet may engage the filter at multiple locations along its length in addition to at the distal tip 41.

An alternative structure for radially collapsing the embolic filter component 11 is shown in Fig. 4 where an outer delivery sheath 50 is placed over the exterior of the cylindrical outer wall 12 of the embolic filter component 11 in order to axially elongate and radially reduce the filter, as shown in full line. By retracting the outer delivery sheath 50 from over the embolic filter component 11, the filter component will reassume the radially expanded configuration, as shown in broken line.

Referring now to Fig. 5A through 5E, delivering of an embolic filter component 11. The aortic anatomy as illustrated in Fig. 5A where an aortic arch AA receives blood flow from an aortic
valve AV in the direction of the arrow so that the blood flows down the descending aorta DA. The brachiocephalic artery BA, the left carotid artery CA, and the left subclavian artery, referred to herein collectively as the "aortic side vessels," all branch from the aortic arch and a primary purpose of the present disclosure is to prevent emboli released from the aortic valve from entering these aortic side vessels during the performance of any aortic valve intervention.

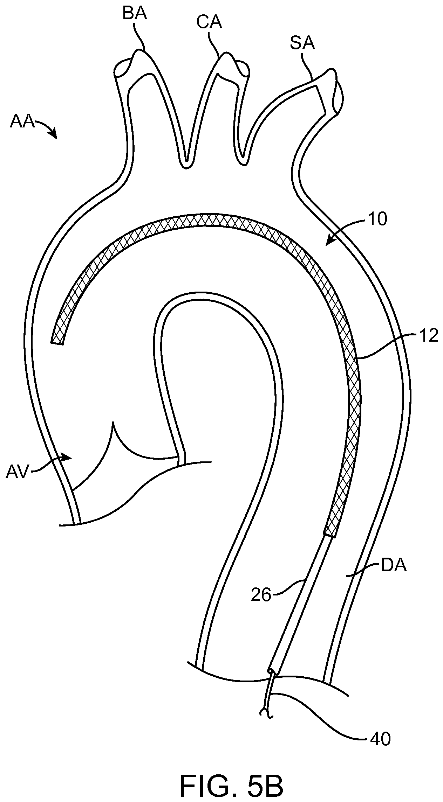

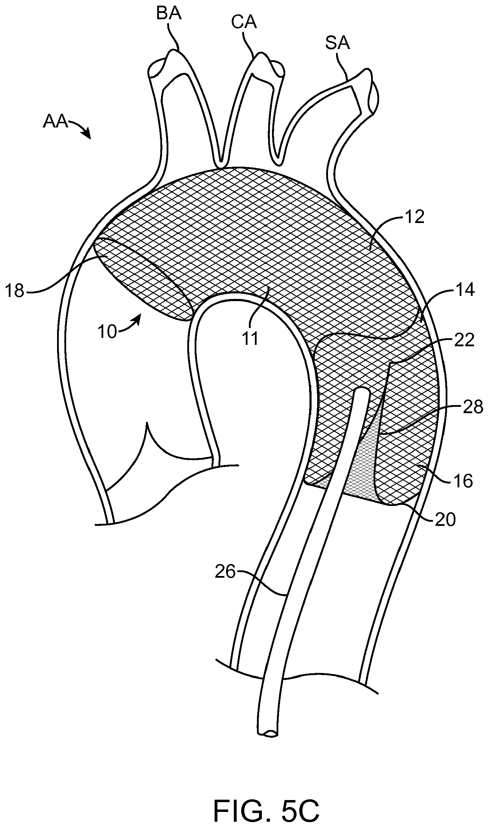

As shown in Fig. 5B, the embolic protection device 10 of Fig. 1 may be advanced using a stylet 40, as illustrated in Fig. 3. Once in place with the cylindrical outer wall 12 disposed over the aortic arch, the stylet 40 may be removed allowing the cylindrical outer wall 12 to radially expand to cover at least the entries into the aortic side vessels, as shown in Fig. 5C. Once the filter is deployed, the open upstream end 18 of the embolic filter component 11 is disposed over the aortic valve AV to provide catheter access for one more interventional catheters. It will be appreciated that this open upstream end 18, while allowing relatively unfettered access for the intervention, will also receive emboli released by the intervention into the interior 14 of the embolic filter component 11. The presence of the porous mesh or other filter structure of the embolic filter component 11 over the entries to the aortic side vessels will divert and prevent emboli from entering these vessels.

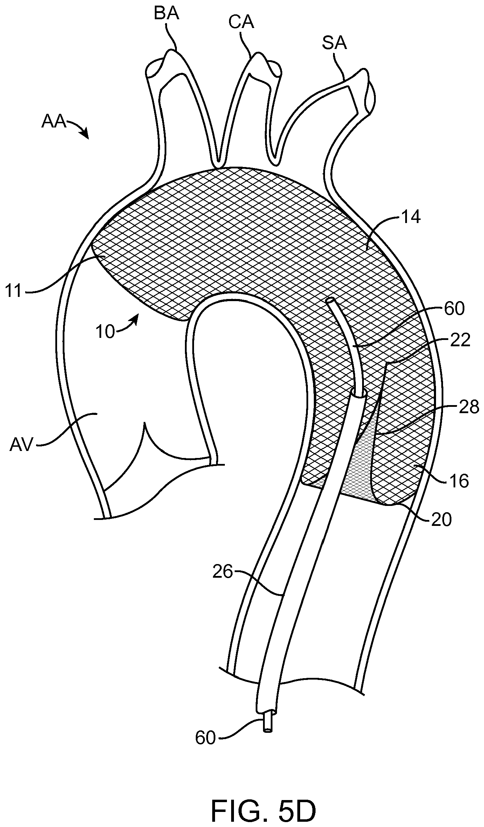

As shown in Fig. 5D, a first catheter, such as a contrast delivery catheter 60, may be introduced through a lumen of the inner sheath 26 so that it enters into the interior 14 of the embolic filter component 11, typically at a location close to the side branch vessels.

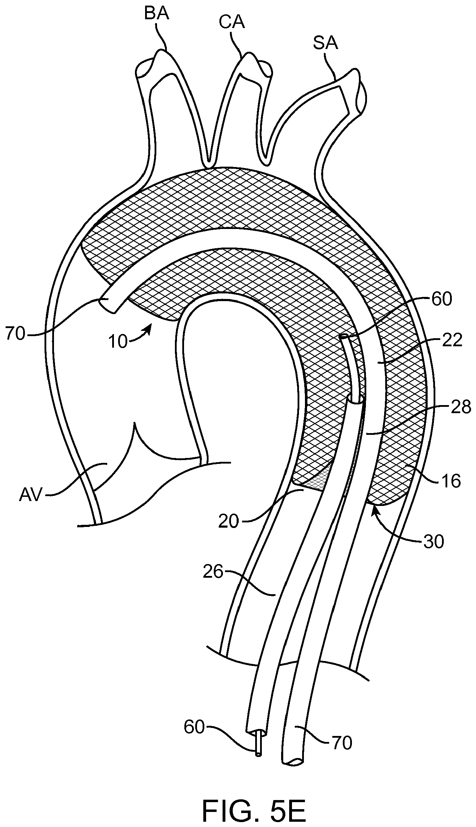

As shown in Fig. 5E, a second catheter 70, typically an interventional catheter such as a valve delivery catheter, an annuloplasty catheter, or the like, may then be introduced through the second port 22 by passing a distal end of the catheter through the wide opening 30 at the base of the first conical inner portion 28 so that the catheter 70 passes through and opens the first port 22. The first port 22 will preferably expand and conform over the exterior of the second catheter 70 so that the risk of emboli passing through the second port is minimized or eliminated. Upon removal of the second catheter 70, the access port 22 will close to prevent emboli from passing through it.

The methods and apparatus of the present disclosure are not limited to any particular interventional or diagnostic catheters or the performance of any particular interventional or diagnostic procedures. Instead, the access ports 22 and 24 can provide for introduction of a wide variety of catheters and tools for performing a number of desired interventions on the aortic valve or anywhere in ascending aorta therein. Further alternative embodiments may include more than two access ports, at least one of which will be expandable as with port 22 and at least one of which will be fixedly attached to an access sheath as with port 24. Additional expandable ports may also include additional inner conical portions.

## Claims

1. An embolic protection device, said device comprising:
an inner sheath (26) having a lumen with a distal opening, wherein the inner lumen is configured to allow a catheter to be advanced therethrough; and
an embolic filter (11) comprising a porous mesh material and having a cylindrical outer wall (12) which defines an interior (14) including a collection chamber (16) for capturing emboli, the filter having an open upstream end (18), a closed downstream end (20), a radially collapsed delivery configuration, and a radially expanded configuration, wherein the outer wall (12) is configured to contact a blood vessel wall to direct blood flow through the upstream end and emboli into the collection chamber (16);
wherein the embolic filter (11) has at least a first port (22) in the closed downstream end (20) thereof comprising an expandable opening configured to conform to an outer wall of a first catheter passing there through, wherein the expandable opening is disposed at an apical end of a first conical inner portion (28) of the enclosed downstream end (20) of the embolic filter (11); and
wherein the inner sheath (26) is attached to a second port (24) in the enclosed downstream end (20) of the embolic filter (11) and to allow a second catheter to be advanced through the lumen of the inner sheath (26) to enter the interior of the embolic filter (11).

2. An embolic protection device as in claim 1, wherein the first conical inner portion (28) defines the collection chamber (16) between an inner surface of the cylindrical outer wall (12) and an outer surface of the conical inner portion (28), wherein the apical end of the conical inner portion is oriented toward the open upstream end (18) of the embolic filter (11), optionally wherein the conical inner portion is positioned inside the cylindrical outer wall (12) and has a wider proximal end joined to the open end of cylindrical outer wall to form the closed downstream end (20).

3. An embolic protection device as in claim 1, wherein the first port (22) further comprising a resilient seal positioned within the first port for forming a seal around a catheter passing through the first port.

4. An embolic protection device as in claim 1, wherein the embolic filter (11) further includes a second conical inner portion (32) which together with the first conical inner portion (28) defines the collection chamber (16) between an inner surface of the cylindrical outer wall (12) and outer surfaces of both the first and second conical inner portions, wherein an apical end of the second conical inner portion is oriented toward the open upstream end (18) of the embolic filter.

5. An embolic protection device as in claim 1, wherein a distal portion of the inner sheath (26) extends through the second port (24) and is attached to an inner surface of the outer cylindrical wall (12) so that the distal opening of the inner sheath (26) lies inside the collection chamber (16).

6. An embolic protection device as in claim 3, wherein a distal portion of the inner sheath (26) extends through the second port (24) and is attached to an outer surface of the first conical portion (28).

7. An embolic protection device as in claim 1, wherein the open upstream end (18) of the embolic filter (11) is configured to allow blood to flow between the first conical inner portion (28) and the cylindrical outer portion (12), with a space between the first conical inner portion (28) and the cylindrical outer portion (12) defining the collection chamber (16) for captured emboli.

8. An embolic protection device as in claim 1, wherein the porous mesh material self-expands into the expanded configuration when the filter (11) is in the deployed configuration.

9. An embolic protection device as in claim 8, wherein the porous mesh material comprises a fabric of knitted, woven, or nonwoven fibers, filaments, or wires having a pore size chosen to prevent emboli over a predetermined size from passing through.

10. An embolic protection device as in claim 8, wherein the porous mesh material is made of a resilient metal, polymer material, a malleable material, a plastically deformable material, a shape-memory material, or combinations thereof.

11. An embolic protection system comprising:
an embolic protection device (10) as in claim 1; and
an outer delivery sheath (50) having a lumen there through configured to restrain the embolic filter (11) in its radially collapsed delivery configuration when the embolic filter is there within, wherein the embolic filter (11) deploys into its radially expanded configuration when the embolic filter (11) is advanced out of the outer delivery sheath (50).

12. An embolic protection system comprising:
an embolic protection device (10) as in claim 1; and
a stylet (40) configured to be advanced through the sheath lumen and the embolic filter (11) to elongate the embolic filter to assume its radially collapsed delivery configuration, wherein the embolic filter deploys into its radially expanded configuration when the stylet (40) is removed from the embolic filter.

## Patentansprüche

1. Eine Embolieschutzvorrichtung, wobei die Vorrichtung Folgendes beinhaltet:
eine innere Schleuse (26), die ein Lumen mit einer distalen Öffnung aufweist, wobei das innere Lumen dazu ausgelegt ist, das Vorschieben eines Katheter dadurch zu erlauben; und
einen Emboliefilter (11), der ein poröses Meshmaterial beinhaltet und eine zylindrische Außenwand (12) aufweist, die ein Inneres (14) definiert, das eine Sammelkammer (16) zum Einfangen von Emboli umfasst, wobei das Filter ein offenes stromaufwärts liegendes Ende (18), ein geschlossenes stromabwärts liegendes Ende (20), eine radial zusammengefaltete Zuführkonfiguration und eine radial expandierte Konfiguration aufweist, wobei die Außenwand (12) dazu ausgelegt ist, mit einer Blutgefäßwand in Berührung zu kommen, um den Blutstrom durch das stromaufwärts liegende Ende zu leiten und Emboli in die Sammelkammer (16) zu leiten;
wobei das Emboliefilter (11) mindestens einen ersten Zugang (22) in dem geschlossenen stromabwärts liegenden Ende (20) davon aufweist, der eine expandierbare Öffnung beinhaltet, die dazu ausgelegt ist, einer Außenwand eines ersten Katheters, der durch diese durchgeht, zu entsprechen, wobei die expandierbare Öffnung an einem apikalen Ende eines ersten konisch zulaufenden inneren Anteild (28) des eingeschlossenen stromabwärts liegenden Endes (20) des Emboliefilters (11) angeordnet ist; und
wobei die innere Schleuse (26) an einem zweiten Zugang (24) in dem eingeschlossenen stromabwärts liegenden Ende (20) des Emboliefilters (11) befestigt ist und um das Vorschieben eines zweiten Katheters durch das Lumen der inneren Schleuse (26) zu erlauben, um in das Innere des Emboliefilters (11) zu gelangen.

2. Embolieschutzvorrichtung nach Anspruch 1, wobei der erste konisch zulaufende innere Anteil (28) die Sammelkammer (16) zwischen einer Innenfläche der zylindrischen Außenwand (12) und einer Außenfläche des konisch zulaufenden inneren Anteils (28) definiert, wobei das apikale Ende des konisch zulaufenden inneren Anteils zu dem offenen stromaufwärts liegenden Ende (18) des Emboliefilters (11) ausgerichtet ist, wobei der konisch zulaufende innere Anteil optional innerhalb der zylindrischen Außenwand (12) positioniert ist und ein weiteres proximales Ende aufweist, das mit dem offenen Ende der zylindrischen Außenwand verbunden ist, um das geschlossene stromabwärts liegende Ende (20) zu bilden.

3. Embolieschutzvorrichtung nach Anspruch 1, wobei der erste Zugang (22) ferner eine elastische Dichtung, die innerhalb des ersten Zugangs positioniert ist, zum Bilden einer Dichtung um einen Katheter, der durch den ersten Zugang durchgeht, beinhaltet.

4. Embolieschutzvorrichtung nach Anspruch 1, wobei das Emboliefilter (11) ferner einen zweiten konisch zulaufenden inneren Anteil (32) umfasst, der zusammen mit dem ersten konisch zulaufenden inneren Anteil (28) die Sammelkammer (16) zwischen einer Innenfläche der zylindrischen Außenwand (12) und den Außenflächen sowohl des ersten als auch des zweiten konisch zulaufenden inneren Anteils definiert, wobei ein apikales Ende des zweiten konisch zulaufenden inneren Anteils zu dem offenen stromaufwärts liegenden Ende (18) des Emboliefilters ausgerichtet ist.

5. Embolieschutzvorrichtung nach Anspruch 1, wobei ein distaler Anteil der inneren Schleuse (26) sich durch den zweiten Zugang (24) hindurch erstreckt und an einer Innenfläche der zylindrischen Außenwand (12) befestigt ist, sodass die distale Öffnung der inneren Schleuse (26) innerhalb der Sammelkammer (16) liegt.

6. Embolieschutzvorrichtung nach Anspruch 3, wobei ein distaler Anteil der inneren Schleuse (26) sich durch den zweiten Zugang (24) erstreckt und an einer Außenfläche des ersten konisch zulaufenden Anteils (28) befestigt ist.

7. Embolieschutzvorrichtung nach Anspruch 1, wobei das offene stromaufwärts liegende Ende (18) des Emboliefilters (11) dazu ausgelegt ist, Blut zwischen dem ersten konisch zulaufenden inneren Anteil (28) und dem zylindrischen äußeren Anteil (12) strömen zu lassen, wobei ein Raum zwischen dem ersten konisch zulaufenden inneren Anteil (28) und dem zylindrischen äußeren Anteil (12) die Sammelkammer (16) für eingefangene Emboli definiert.

8. Embolieschutzvorrichtung nach Anspruch 1, wobei das poröse Meshmaterial sich selbst in die expandierte Konfiguration expandiert, wenn das Filter (11) in der abgelegten Konfiguration ist.

9. Embolieschutzvorrichtung nach Anspruch 8, wobei das poröse Meshmaterial ein Gewebe aus gewirkten, gewebten oder nicht gewebten Fasern, Filamenten oder Drähten mit einer Porengröße beinhaltet, die so gewählt ist, dass das Durchtreten von Emboli über einer vorbestimmten Größe verhindert wird.

10. Embolieschutzvorrichtung nach Anspruch 8, wobei das poröse Meshmaterial aus einem elastischen Metall, Polymermaterial, einem umformbaren Material, einem plastisch verformbaren Material, einem Formgedächtnismaterial oder Kombinationen davon hergestellt ist.

11. Ein Embolieschutzsystem, das Folgendes beinhaltet:
eine Embolieschutzvorrichtung (10) nach Anspruch 1; und
eine äußere Zuführschleuse (50) mit einem dadurch verlaufenden Lumen, die dazu ausgelegt ist, das Emboliefilter (11) in seiner radial zusammengefalteten Zuführkonfiguration zu halten, wenn das Emboliefilter sich in ihrem Inneren befindet, wobei das Emboliefilter (11) in seiner radial expandierten Konfiguration abgelegt wird, wenn das Emboliefilter (11) aus der äußeren Zuführschleuse (50) heraus vorgeschoben wird.

12. Ein Embolieschutzsystem, das Folgendes beinhaltet:
eine Embolieschutzvorrichtung (10) nach Anspruch 1; und
einen Mandrin (40), der dazu ausgelegt ist, durch das Schleusenlumen und das Emboliefilter (11) vorgeschoben zu werden, um das Emboliefilter zu verlängern, um seine radial zusammengefaltete Zuführkonfiguration anzunehmen, wobei das Emboliefilter in seiner radial expandierten Konfiguration abgelegt wird, wenn der Mandrin (40) aus dem Emboliefilter entfernt wird.

## Revendications

1. Dispositif à protection embolique, ledit dispositif comprenant :
une gaine intérieure (26) comportant une lumière ayant une ouverture distale, la lumière intérieure étant configurée pour permettre de faire avancer un cathéter dans ladite lumière ; et
un filtre embolique (11) comprenant un matériau sous forme de maille poreuse et comportant une paroi extérieure cylindrique (12) qui délimite un intérieur (14) comprenant une chambre de collecte (16) pour retenir des emboles, le filtre ayant une extrémité amont ouverte (18), une extrémité aval fermée (20), une configuration de mise en place radialement affaissée, et une configuration radialement expansée, la paroi extérieure (12) étant configurée pour entrer en contact avec une paroi d'un vaisseau sanguin pour faire passer l'écoulement sanguin par l'extrémité amont et amener les emboles dans la chambre de collecte (16) ;
dans lequel le filtre embolique (11) comporte au moins un premier orifice (22) dans l'extrémité aval fermée (20) dudit filtre, comprenant une ouverture expansible configurée pour se conformer à une paroi extérieure d'un premier cathéter passant dans ladite ouverture, l'ouverture expansible étant disposée à une extrémité apicale d'une première partie intérieure conique (28) de l'extrémité aval fermée (20) du filtre embolique (11) ; et
dans lequel la gaine intérieure (26) est fixée à un deuxième orifice (24) situé dans l'extrémité aval fermée (20) du filtre embolique (11) et permettant de faire avancer un deuxième cathéter dans la lumière de la gaine intérieure (26) de manière à ce qu'il pénètre à l'intérieur du filtre embolique (11).

2. Dispositif à protection embolique selon la revendication 1, dans lequel la première partie intérieure conique (28) délimite la chambre de collecte (16) entre une surface intérieure de la paroi extérieure cylindrique (12) et une surface extérieure de la partie intérieure conique (28), dans lequel l'extrémité apicale de la partie intérieure conique est orientée vers l'extrémité amont ouverte (18) du filtre embolique (11), optionnellement dans lequel la partie intérieure conique est positionnée à l'intérieur de la paroi extérieure cylindrique (12) et comporte une extrémité proximale plus large jointe à l'extrémité ouverte de la paroi extérieure cylindrique pour former l'extrémité aval fermée (20).

3. Dispositif à protection embolique selon la revendication 1, dans lequel le premier orifice (22) comprend en outre un joint résilient positionné dans le premier orifice pour former un joint étanche autour d'un cathéter passant dans le premier orifice.

4. Dispositif à protection embolique selon la revendication 1, dans lequel le filtre embolique (11) comprend en outre une deuxième partie intérieure conique (32) qui, conjointement avec la première partie intérieure conique (28), délimite la chambre de collecte (16) entre une surface intérieure de la paroi extérieure cylindrique (12) et les surfaces extérieures de la première partie intérieure conique ainsi que de la deuxième partie intérieure conique, dans lequel une extrémité apicale de la deuxième partie intérieure conique est orientée vers l'extrémité amont ouverte (18) du filtre embolique.

5. Dispositif à protection embolique selon la revendication 1, dans lequel une partie distale de la gaine intérieure (26) s'étend à travers le deuxième orifice (24) et est fixée à une surface intérieure de la paroi cylindrique extérieure (12) de telle sorte que l'ouverture distale de la gaine intérieure (26) se trouve à l'intérieur de la chambre de collecte (16).

6. Dispositif à protection embolique selon la revendication 3, dans lequel une partie distale de la gaine intérieure (26) s'étend à travers le deuxième orifice (24) et est fixée à une surface extérieure de la première partie conique (28).

7. Dispositif à protection embolique selon la revendication 1, dans lequel l'extrémité amont ouverte (18) du filtre embolique (11) est configurée pour permettre au sang de s'écouler entre la première partie intérieure conique (28) et la partie extérieure cylindrique (12), un espace entre la première partie intérieure conique (28) et la partie extérieure cylindrique (12) délimitant la chambre de collecte (16) pour les emboles retenus.

8. Dispositif à protection embolique selon la revendication 1, dans lequel le matériau sous forme de maille poreuse se déploie de lui-même pour prendre la configuration expansée quand le filtre (11) est dans la configuration déployée.

9. Dispositif à protection embolique selon la revendication 8, dans lequel le matériau sous forme de maille poreuse comprend un tissu tricoté, tissé, ou des fibres, des filaments ou des fils métalliques non tissés ayant une taille de pore choisie pour empêcher des emboles au-dessus d'une taille prédéterminée de passer à travers.

10. Dispositif à protection embolique selon la revendication 8, dans lequel le matériau sous forme de maille poreuse se compose d'un métal résilient, d'un matériau polymère, d'un matériau malléable, d'un matériau capable de subir une déformation plastique, d'un matériau à mémoire de forme, ou de combinaisons de ceux-ci.

11. Système à protection embolique comprenant :
un dispositif à protection embolique (10) selon la revendication 1 ; et
une gaine de mise en place extérieure (50) à travers laquelle s'étend une lumière configurée pour maintenir le filtre embolique (11) dans sa configuration de mise en place radialement affaissée quand le filtre embolique se trouve à l'intérieur de ladite lumière, le filtre embolique (11) se déployant dans sa configuration radialement expansée quand on fait avancer le filtre embolique (11) de manière à ce qu'il sorte de la gaine de mise en place extérieure (50).

12. Système à protection embolique comprenant :
un dispositif à protection embolique (10) selon la revendication 1 ; et
un stylet (40) configuré pour qu'on le fasse avancer dans la lumière de la gaine et le filtre embolique (11) afin d'allonger le filtre embolique pour qu'il prenne sa configuration de mise en place radialement affaissée, le filtre embolique se déployant dans sa configuration radialement expansée quand le stylet (40) est retiré du filtre embolique.
